Europäisches Patentamt
European Patent Office
Office européen des brevets

(19) (11) Publication number: **0 083 737 A1**

# EUROPEAN PATENT APPLICATION

(21) Application number: 82111332.1

(22) Date of filing: 07.12.82

(51) Int. Cl.$^3$: **C 07 D 451/14**
C 07 D 451/04, A 61 K 31/435
//C07C65/21

(30) Priority: 15.12.81 GB 8137821
03.07.82 GB 8219296

(43) Date of publication of application: 20.07.83 Bulletin 83/29

(84) Designated Contracting States: BE CH DE FR GB IT LI NL SE

(71) Applicant: BEECHAM GROUP PLC
Beecham House Great West Road
Brentford Middlesex(GB)

(72) Inventor: Hadley, Michael Stewart
9 Coney Gree
Sawbridgeworth Hertfordshire(GB)

(72) Inventor: Hunter, David James
17 Park Lane
Bishops Stortford Hertfordshire(GB)

(74) Representative: Stott, Michael John et al,
European Patent Attorney Beecham Pharmaceuticals
Great Burgh Yew Tree Bottom Road
Epsom Surrey KT18 5XQ(GB)

(54) **N-azabicycloalkane benzamides, process for their preparation and pharmaceutical compositions containing them.**

(57) Compounds of formula (I) and pharmaceutically acceptable salts thereof:

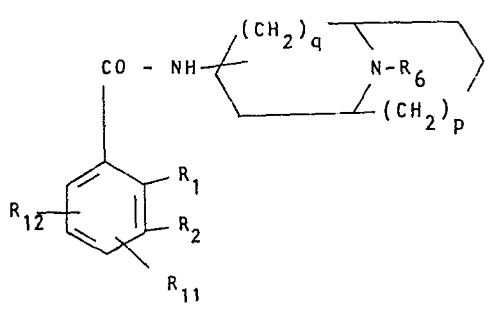

(I)

wherein:

p is 1 or 2;

q is 0 to 2;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-4}$ alkyl, $C_{1-4}$ alkoxy, trifluoromethyl, halogen or nitro, or a thienyl group.

$R_{12}$ is hydrogen, halogen, $CF_3$, $C_{1-7}$ acyl, $C_{1-7}$ acylamino or amino or aminocarbonyl optionally substituted by one or two $C_{1-6}$ alkyl groups, $C_{1-6}$ alkyl, $C_{1-6}$ alkoxy, hydroxy or nitro;

$R_1$ is $C_{1-6}$ alkoxy or $C_{1-6}$ alkylthio; and one of $R_2$ and $R_{11}$ is hydrogen and the other is $C_{1-6}$ alkoxy, $C_{1-6}$ alkyl or hydroxyl;

or $R_1$ and $R_2$ together form methylenedioxy or ethylenedioxy; and

$R_{11}$ is any one of the groups given for $R_{12}$ above having dopamine antagonist activity, a process for their preparation and their use.

EP 0 083 737 A1

0083737

PJ/B1176/1288

TITLE MODIFIED
see front page

ACTIVE COMPOUNDS

This invention relates to novel compounds, to pharmaceutical compositions containing them, and to a process for their preparation.

European Patent Application No. 79302978.6 discloses that compounds of the formula (A), and pharmaceutically acceptable salts thereof:

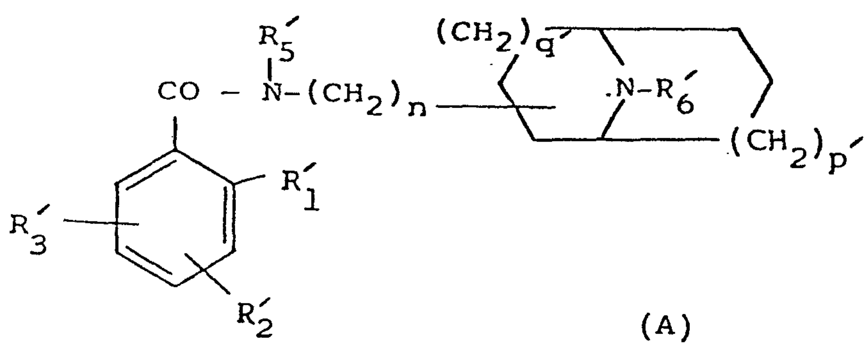

(A)

(A)

wherein:

$R_1'$ is a $C_{1-6}$ alkoxy group;

$R_2'$ and $R_3'$ are the same or different and are hydrogen, halogen, $CF_3$, $C_{2-7}$ acyl, $C_{2-7}$ acylamino, or amino, aminocarbonyl or aminosulphone optionally substituted by one or two $C_{1-6}$ alkyl groups, $C_{1-6}$ alkylsulphone or nitro;

$R_5'$ is hydrogen or $C_{1-6}$ alkyl;

$R_6'$ is $C_{1-7}$ alkyl or a group $-(CH_2)_sR_7'$ where s is 0 to 2 and $R_7'$ is a $C_{3-8}$ cycloalkyl group, or a group $-(CH_2)_{t'}R_8$ where t' is 1 or 2 and $R_8'$ is $C_{2-5}$ alkenyl or a phenyl group optionally substituted by one or two substituents selected from $C_{1-6}$ alkyl, $C_{1-4}$ alkoxy, trifluoromethyl and halogen; and

n, p' and q' are independently 0 to 2; have useful pharmacological activity. More specifically the compounds of formula (A) are stated to be useful in the treatment of disorders related to impaired gastro-intestinal motility and/or in the treatment of disorders of the central nervous system. All the compounds are stated to have anti-emetic activity.

Australian Patent Application No. 54610/80 discloses compounds of formula (B):

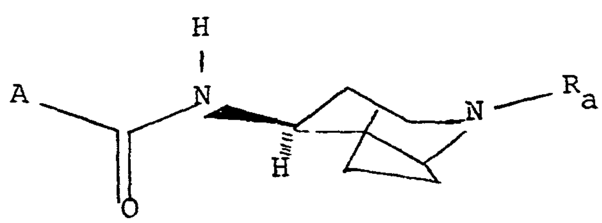

(B)

wherein _inter alia_ $R_a$ is a benzyl nucleus and A _inter alia_ represents:

(i) an aroyl nucleus of formula:

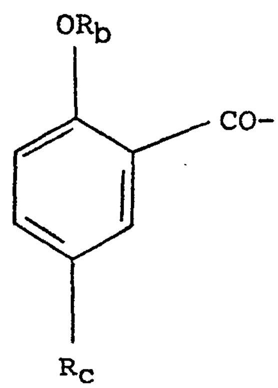

wherein ($R_b$, $R_c$) assumes <u>inter alia</u> the value ($CH_3$, $CH_3O$).

ii) an aroyl nucleus of formula:

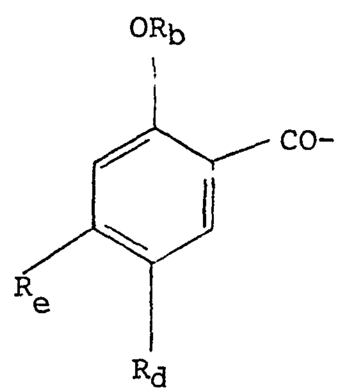

in which the set ($R_b$, $R_d$, $R_e$) assumes <u>inter alia</u> the following values: ($CH_3$, $CH_3O$, $CH_3O$), ($CH_3$, $CH_3O$, H) or

(iii) an aroyl nucleus monosubstituted or polysubstituted by the following groups or atoms : methoxy-3; methoxy-4; chloro-3; chloro-4; fluoro-4; nitro-3; methyl-4; dimethoxy-2,3; dimethoxy-2,6; dimethoxy 3,5; methylenedioxy-3,4; dinitro-3,5;

trimethoxy-2,3,4; trimethoxy-3,4,5; methoxy-2 bromo-5; methoxy-2 dibromo-3,5 amino-4.

The compounds are described as having neuroleptic activity.

It has now been discovered that a group or compounds structurally distinct from those of formulae (A)and (B) also have useful pharmacological activity, namely dopamine antagonist activity.

Accordingly, the present invention provides a compound of the formula (I), and pharmaceutically acceptable salts thereof:

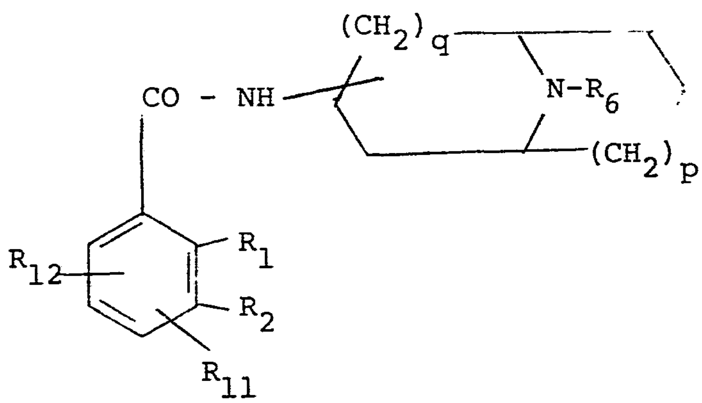

(I)

wherein:

p is 1 or 2;

q is 0 to 2;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-4}$ alkyl, $C_{1-4}$ alkoxy, trifluoromethyl, halogen or nitro, or a thienyl group;

$R_{12}$ is hydrogen, halogen, $CF_3$, $C_{1-7}$ acyl, $C_{1-7}$ acylamino or amino or aminocarbonyl optionally substituted by one or two $C_{1-6}$ alkyl groups, $C_{1-6}$ alkyl, $C_{1-6}$ alkoxy, hydroxy or nitro;
and either

$R_1$ is $C_{1-6}$ alkoxy or $C_{1-6}$ alkylthio; and one of $R_2$ and $R_{11}$ is hydrogen and the other is $C_{1-6}$ alkoxy, $C_{1-6}$ alkyl or hydroxyl;

or $R_1$ and $R_2$ together form methylenedioxy or ethylenedioxy; and

$R_{11}$ is any one of the groups given for $R_{12}$ above.

Suitable examples of $R_6$ when $C_{1-7}$ alkyl include methyl, ethyl, n- and iso-propyl, n, sec and tert-butyl, n-pentyl, n-hexyl and n-heptyl, 3-methylbutyl, 4-methylpentyl and 5-methylhexyl.

When $R_6$ is a group $-(CH_2)_sR_7$ as defined, suitable examples of $R_7$ include $C_{5-8}$ cycloalkyl, preferably cyclohexyl. s is preferably 1.

When $R_6$ is a group $-(CH_2)_t R_8$ as defined, t is preferably 1.

In such a group $R_6$, when $R_8$ is $C_{2-5}$ alkenyl, suitable examples thereof include vinyl, prop-1-enyl, prop-2-enyl, 1-methylvinyl, but-1-enyl, but-2-enyl, but-3-enyl, 1-methylenepropyl, 1-methylprop-1-enyl and 1-methylprop-2-yl, in their E and Z forms where stereoisomerism exists.

A preferred $C_{2-5}$ alkenyl $R_8$ radical is vinyl, so that $R_6$ is preferably allyl.

When $R_8$ is optionally substituted phenyl as defined above, suitable examples of such optional phenyl substituents include methyl, ethyl, n- and iso-propyl, n, sec- and tert- butyl; methoxy, ethoxy, n- and iso-propoxy; $CF_3$, fluoro, chloro, bromo. Favourably $R_8$ when optionally substituted phenyl is unsubstituted or para substituted by $C_{1-4}$ alkyl or halogen.

When $R_8$ is thienyl it may be 2- or 3-thienyl, generally 2-thienyl.

Two values for $R_6$ are optionally substituted benzyl as hereinbefore defined and thienylmethyl (also called thenyl). Optionally substituted benzyl is favoured, preferably benzyl or 4-monosubstituted benzyl.

Suitable examples of the group $R_{12}$ include the following groups: hydrogen chloro, bromo, $CF_3$, amino or aminocarbonyl optionally substituted by inert two methyl groups, formylamino, $C_{1-4}$ alkanoylamino such as acetylamino, propionylamino, n- and iso-butyrylamino, methyl, ethyl, n and iso-propyl, nitro, methoxy, ethoxy, n-and iso- propoxy, and hydroxy.

Particularly suitable $R_{12}$ groups include hydrogen, halogen, $CF_3$ and methoxy.

Suitable examples of the group $R_1$ when $C_{1-6}$ alkoxy or $C_{1-6}$ alkylthio include methoxy, ethoxy and n- and iso-propoxy, methylthio, ethylthio, and n- and iso-propylthio. Preferably $R_1$ is a methoxy group.

Suitable examples of $R_2/R_{11}$ when other than hydrogen include methoxy, ethoxy, n- and iso-propoxy, methyl, ethyl, n- and iso-propyl and hydroxy.

Preferred $R_2/R_{11}$ groups then include methoxy.

When $R_1$ and $R_2$ together are methylenedioxy or ethylenedioxy they are preferably ethylenedioxy.

Suitable examples for $R_{11}$ then include those listed for $R_{12}$ above. $R_{11}$ is often $C_{1-6}$ alkoxy or hydrogen, preferably methoxy or hydrogen.

It is generally preferred that $R_{11}$ when other than $C_{1-6}$ alkoxy is in the 4-position as previously defined.

q is suitably 0 or 1.

p is preferably 1.

Often the amide and heterobicycle nitrogen atoms, which are in the same side chain, are separated by 2 or 3 carbon atoms, most preferably 3.

In such most preferable cases the -CO-NH-moiety is in a β-orientation to the heterobicycle ring.

The pharmaeutically acceptable salts of the compound of the formula (I) include acid addition salts with conventional acids such as hydrochloric, hydrobromic, phosphoric, sulphuric, citric, tartaric, lactic and acetic acid; and quarternary ammonium salts. Examples of such salts include such compounds quaternised by compounds such as $R_9$ - Y wherein $R_9$ is $C_{1-6}$ alkyl, phenyl-$C_{1-6}$ alkyl or $C_{5-7}$ cycloalkyl, and Y is a radical corresponding to an anion of an acid.

Suitable examples of $R_9$ include methyl, ethyl and n- and iso-propyl; and benzyl and phenylethyl. Suitable examples of Y include the halides such as chloride, bromide and iodide.

Examples of pharmaceutically acceptable salts also include internal salts such as N-oxides.

The compounds of the formula (I) may also form solvates such as hydrates and the invention extends to such solvates.

There is a group of compounds within formula (I) wherein the compound of formula (I) is other than 2,3-dimethoxy-N-[9-benzyl-9-azabicyclo [3.3.1] nonan-3β-yl] benzamide or 2-ethoxy-3-methoxy-[9-benzyl-9-azabicyclo [3.3.1]nonan-3β-yl] benzamide.

From the aforesaid it will be seen that favourably the moiety of formula (II):

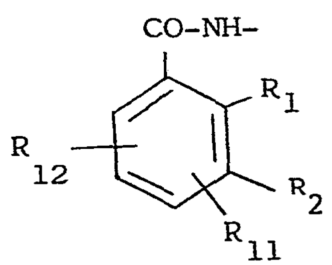

(II)

in a compound of the formula (I) will have the structure (III):

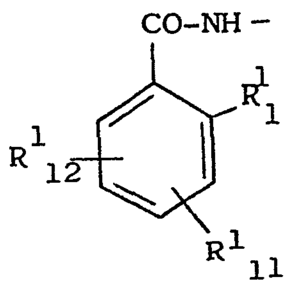

(III)

wherein:

$R_1{}^1$ is $C_{1-6}$ alkoxy or $C_{1-6}$ alkylthio;

one of $R_{11}{}^1$ and $R_{12}{}^1$ is $C_{1-6}$ alkoxy and the other is $C_{1-6}$ alkoxy or hydrogen.

Suitable and preferred values for $R_{12}{}^1$ are as described under formula (I), preferably hydrogen, and $R_{11}{}^1$ and $R_1{}^1$ are then preferably both methoxy.

In particular $R_1{}^1$ and $R_{11}{}^1$ are 2,3- or 2,4 dimethoxy and $R_{12}{}^1$ is hydrogen.

A favourable group of compounds within formula (I) is therefore of formula (IV):

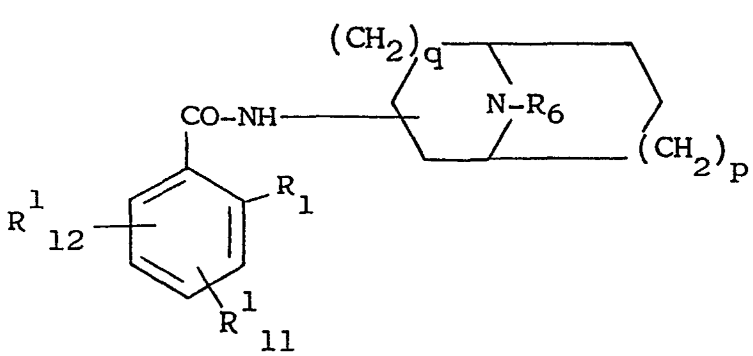

(IV)

0083737

wherein the variable groups are as defined in formula (I), in particular formula (III).

Suitable and preferred values for q and p are as hereinbefore described under formula (I). Preferably p and q are 1 and the moiety of formula (II) is then attached at the 3-position numbering through the $(CH_2)_q$ -containing ring from a bridgehead atom taken as 1 (not necessarily standard numbering).

Suitable and preferred examples of $R_6$ in formula (IV) include those listed under formula (I) for $R_6$.

A sub-group of compounds within those of formula (IV) are those of the formula (V):

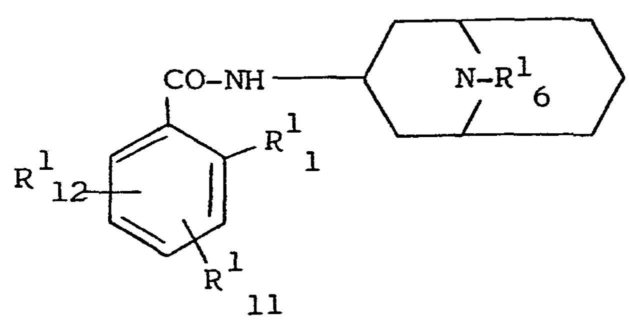

(V)

wherein:

$R_6{}^1$ is $C_{1-7}$ alkyl and the remaining groups are as hereinbefore defined.

Suitable examples of $R_6{}^1$ are as so described for $R_6$ $C_{1-7}$ alkyl groups under formula (I).

It is preferred that the CONH moiety is in the β-orientation to the granatane ring.

A preferred sub-group of compounds within those of formula (IV) are those of formula (VI):

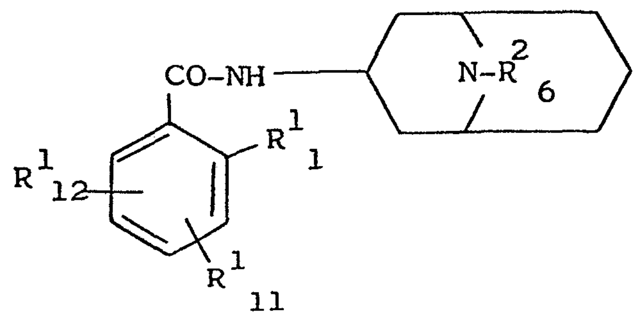

(VI)

wherein:

$R_6{}^2$ is a group $-(CH_2)_tR_7{}^1$ wherein t is 1 or 2 and $R_7{}^1$ is optionally substituted phenyl as defined in formula (I); cyclohexylmethyl; or 2-thienylmethyl; and the remaining variables are as hereinbefore defined.

Suitable and preferred values for $R_1{}^1$, $R_{11}{}^1$, $R_{12}{}^1$ and $R_6{}^2$ are as described for the corresponding variables in formula (I) and (III).

A particularly preferred sub-group of compounds within formula (VI) is of formula (VII):

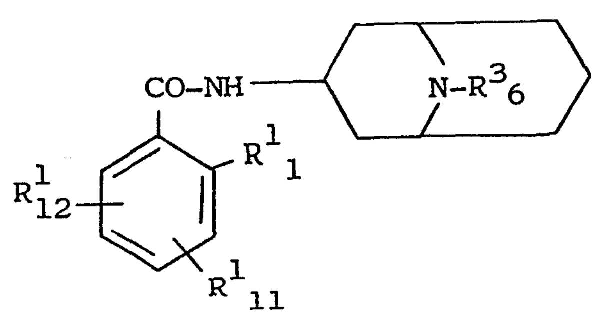

(VII)

wherein $R_6{}^3$ is benzyl para substituted by $C_{1-4}$ alkyl or halogen and the remaining variables are as hereinbefore defined.

Preferred values for $R_6{}^3$ include p-methylbenzyl, p-chlorobenzyl and p-fluorobenzyl.

Suitable preferred and particular values for $R_1{}^1$, $R_{11}{}^1$ and $R_{12}{}^1$ are as described under formula (III).

A further sub-group of compounds within those of formula (IV) which is of interest is of the formula (VIII):

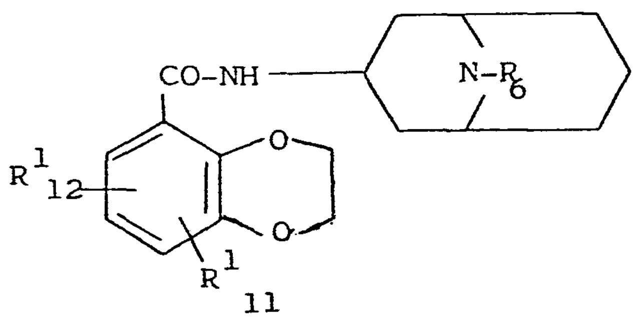

(VIII)

wherein the variables are as hereinbefore defined.

Suitable values for $R_{12}{}^1$ and $R_{11}{}^1$ include hydrogen and methoxy.

Suitable and preferred values for $R_6$ are as described under formula (I).

There are sub-groups of compounds within each of formulae (IV) and (VI) wherein the compound of formula (IV) and (VI) is other than 2,3-dimethoxy-N-[9-benzyl-9-aza-bicyclo [3.3.1]nonan-3β-yl] benzamide or 2-ethoxy-3-methoxy-N-[9-benzyl-9-azabicyclo [3.3.1]nonan-3β-yl] benzamide.

Another favourable group of compounds within formula (I) is of formula (IX):

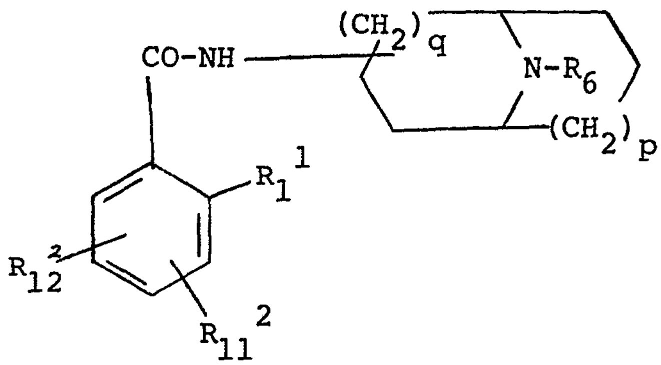

(IX)

wherein one of $R_{11}^2$ and $R_{12}^2$ is $C_{1-6}$ alkoxy and the other is halogen or $CF_3$; and the remaining variables are as hereinbefore defined.

Favourable values for $R_{11}^2$ and $R_{12}^2$ are methoxy and chloro or bromo. Preferred values are 3-methoxy and 5- or 6-chloro, most preferably 3-methoxy-6-chloro.

Suitable and preferred values of the remaining variable groups are as described under formulae (I) and (IV).

There are three sub-groups within formula (IX) each having a benzamide nucleus as defined in formula (IX) and a side chain as defined in formula (V), (VI) and (VII) respectively.

Within the second of these sub-groups there is a preferred sub-group of the formula (X):

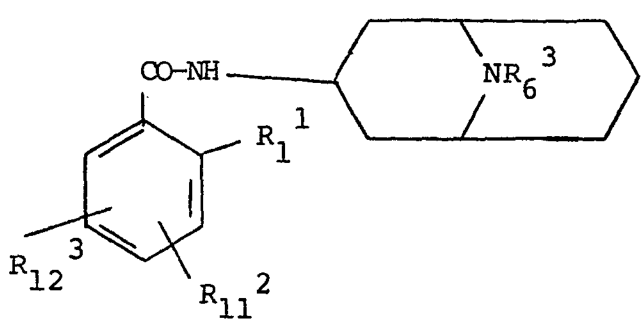

(X)

wherein the variable groups are as defined in formula (X).

Preferred values for $R_6{}^3$ are as described in formula (VII).

Suitable and preferred values for $R_1{}^1$, $R_{11}{}^2$ and $R_{12}{}^2$ are as described in formula (IX).

Particularly suitable examples of the compounds of the present invention include those of the Examples hereinafter.

It will of course be realised that the compounds of the formula (I) have chiral or prochiral centres, and thus are capable of existing in a number of stereoisomeric forms, and to mixtures thereof (including racemates). The different stereoisomeric forms may be separated one from the other by the usual mthods, or any given isomer may be obtained by stereospecific or asymmetric synthesis.

The invention also provides a process for the preparation of a compound of the formula (I), which process comprises reacting a compound of the formula (XI):

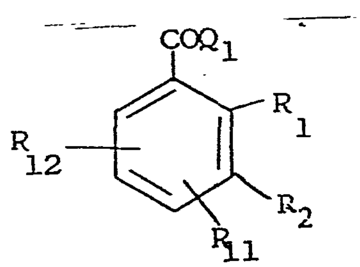

(XI)

wherein $Q_1$ is a leaving group with a compound of formula (XII):

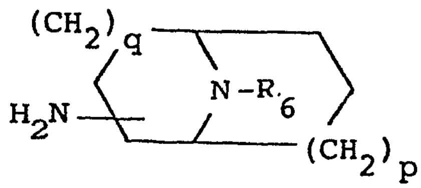

(XII)

wherein the variables are as defined in formula (I), and thereafter if desired or necessary converting a group $R_2$, $R_{11}$ or $R_{12}$ in the thus formed compound to another group $R_2$, $R_{11}$ or $R_{12}$ respectively; as necessary converting $R_6$ to (other) $R_6$; and optionally forming a pharmaceutically acceptable salt of the resultant compound of the formula (I).

The leaving group $Q_1$ is a group readily displaceable by a nucleophile. Suitable examples of Q are hydroxy, halogen such as chloro and bromo and acyloxy such as $C_{1-4}$ alkoxycarbonyloxy or $C_{1-4}$ hydrocarbyloxy such as pentachlorophenoxy.

If the leaving group is hydroxy, then the reaction is preferably carried out in an inert non-hydroxylic solvent, such as benzene, toluene, dichloromethane diethyl ether in the presence of a dehydrating catalyst, such as a carbodiimide, for example dicylohexylcarbodiimide. The reaction may be carried out at a non-extreme temperature such as -10 to 100°C, for example 0 to 80°C.

If the leaving group is a halide, then the reaction is preferably carried out at a non-extreme temperature in an inert non-hydroxylic solvent, such as benzene, toluene or diethyl ether. It is also preferably carried out in the presence of an acid acceptor, such as an organic base, in particular a

tertiary amine, such as triethylamine, pyridine or picoline, some of which can also function as the solvent. Alternatively, the acid acceptor can be inorganic, such as calcium carbonate, sodium carbonate or potassium carbonate.

If the leaving group is acyloxy, then the reaction is preferably carried out in substantially the same manner as if the leaving group were hydroxy. Suitable examples of acyloxy leaving groups include $C_{1-4}$ alkanoyloxy, mesyloxy, tosyloxy and triflate.

If the leaving group is $C_{1-4}$ alkoxycarbonyloxy, then the reaction is preferably carried out in an inert solvent, such as methylenechloride, at a non-extreme temperature in the presence of an acid acceptor, such as triethylamine.

If the leaving group is activated hydrocarbyloxy then the reaction is preferably carried out in an inert polar solvent, such as dimethylformamide. It is also preferred that the activated hydrocarbyloxy is a pentyachlorophenyl ester and that the reaction is carried out at ambient temperature.

Preferably $Q_1$ is hydroxy, chloro or, in particular, $C_{1-4}$ alkoxycarbonyloxy, such as ethoxycarbonyloxy.

The skilled man will appreciate that the choice or necessity of conversion of groups $R_{11}$ and/or $R_{12}$ to other groups $R_{11}$ and/or $R_{12}$ will be dictated by the nature and position of substituents $R_1$, $R_2$, $R_{11}$ and $R_{12}$.

It will be apparent that compounds of the formula (I) containing an $R_{11}$ or $R_{12}$ group which is convertible to another $R_{11}$ or $R_{12}$ group are useful novel intermediates.

By way of example of such conversions, the compounds of the formula (I) wherein $R_{11}$ or $R_{12}$ is a nitro group may be prepared via nitration. For the preparation of the compounds of the formula (I) wherein $R_{11}$ or $R_{12}$ is an amino group, the reduction of a corresponding intermediate wherein $R_{11}$ or $R_{12}$ is a nitro group, may be employed.

The reduction of the intermediates wherein $R_{11}$ or $R_{12}$ is a nitro group may be effected with reagents known to be suitable for reducing nitroanisole to aminoanisole.

In general howwever, it is more convenient to prepare a compound of the formula (I) wherein $R_{11}$ or $R_{12}$ is an amino group from the corresponding $C_{1-7}$ acylamino acid or its reactive derivative, and to deacylate the compound of the formula (I) so formed.

Those compounds of the invention wherein $R_{11}$ or $R_{12}$ is a $C_{1-7}$ acylamino group may be prepared from the corresponding intermediate wherein $R_{11}$ or $R_{12}$ is an amino group by reaction with an acylating derivative, such as previously described as a suitable acylating derivative, e.g. of the acid of the formula (XI). The reaction may proceed as described for the reaction of the compounds of the formula (XI) and (XII). For an $R_{11}/R_{12}$ formamido group acylation may be effected with the free acid.

This invention thus also provides an optional process for the preparation of a compound of the formula (I) wherein $R_{11}$ or $R_{12}$ is an amino group which process comprises the deacylation of a corresponding intermediate wherein $R_{11}$ or $R_{12}$ is a $C_{1-7}$ acylamino group.

Generally the hydrolysis reaction may be effected by treatment with a base such as an alkali metal hydroxide.

Also a compound of the formula (I) wherein $R_{11}$ or $R_{12}$ is halogen may be prepared by a conventional halogenation of the corresponding intermediate wherein the said $R_{11}$ or $R_{12}$ is hydrogen.

It will be appreciated that, $R_6$, when optionally substituted benzyl as hereinbefore defined, may be replaced by another group $R_6$.

Such $R_6$ benzyl may be removed for example when $R_{11}$ or $R_{12}$ is not halogen by conventional transition metal catalysed hydrogenolysis to give compounds of the formula (XIII):

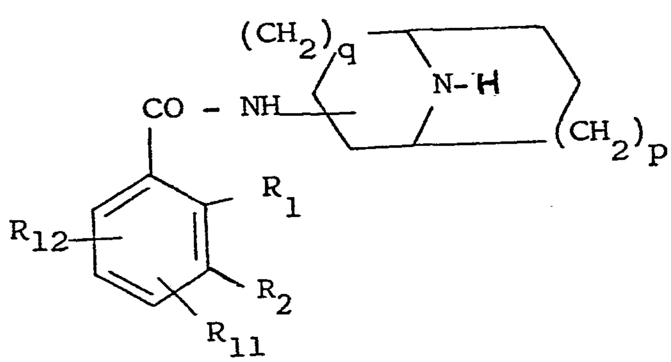

(XIII)

wherein the variable groups are as defined in formula (I).

This invention also provides an optional process step in the preparation of a compound of the formula (I) which comprises the reaction of a corresponding compound of the formula (XIII) as hereinbefore defined with a compound $Q_2R_6$ wherein $R_6$ is as defined in formula (I) and $Q_2$ is a group or atom readily displaced by a nucleophile, and optionally forming a pharmaceutically acceptable salt of the resulting compound of the formula (I).

Suitable values for $Q_2$ include Cl, Br, I, $OSO_2CH_3$ or $OSO_2C_6H_4pCH_3$.

Favoured values for $Q_2$ include Cl, Br and I.

The reaction may be carried out under conventional alkylation conditions for example in an inert solvent such as dimethylformamide in the presence of an acid acceptor such as potassium carbonate. Generally the reaction is carried out at a non-extreme temperature such as at ambient or at a slightly elevated temperature.

$R_6$ interconversion in the compound of the formula (XII) before coupling with the compound of the formula (XI) or its derivative is preferred. Such interconversions are effected conveniently under the above conditions. It is desirable to protect the amine function with a group readily removable by acidolysis such as a $C_{2-7}$ alkanoyl group before $R_6$ interconversion.

The acid addition salts of compounds of the formula (I) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_8Y$ as defined. This reaction is suitably carried out in an appropriate solvent such as acetone or methanol

The nitrogen atom of the heterocyclic side chain may also form an N-oxide to give an internal N-oxide salt of the compound of the formula (I). The N-oxides may be prepared in conventional manner such as by reaction of the chosen compound of the formula (I) with an organic peracid, such as m-chloroperbenzoic acid. This reaction is suitably carried out at below-ambient temperature in an organic solvent, preferably a chlorinated hydrocarbon.

It will be realised that in the compound of the formula (I) the -CO-NH- linkage may have an α or β orientation with respect to the ring of the bicyclic moiety to which it is attached. A mixture of α and β isomers of the compound of the formula (I) may be synthesised non-stereospecifically and the desired isomer separated conventionally therefrom eg by chromatography; or alternatively the α and β isomer may if desired be synthesised from the corresponding α or β form of the compound of the formula (XII).

Synthesis from the corresponding α or β isomer of the compound of the formula (XII) is in general preferred.

The α or β form of the compound of the formula (XII) may if desired be prepared by known stereospecific processes, such as those leading to the α or β isomers of the compound of the formula (XII) depicted in the Schemes and described in the Descriptions hereinafter.

Scheme 1

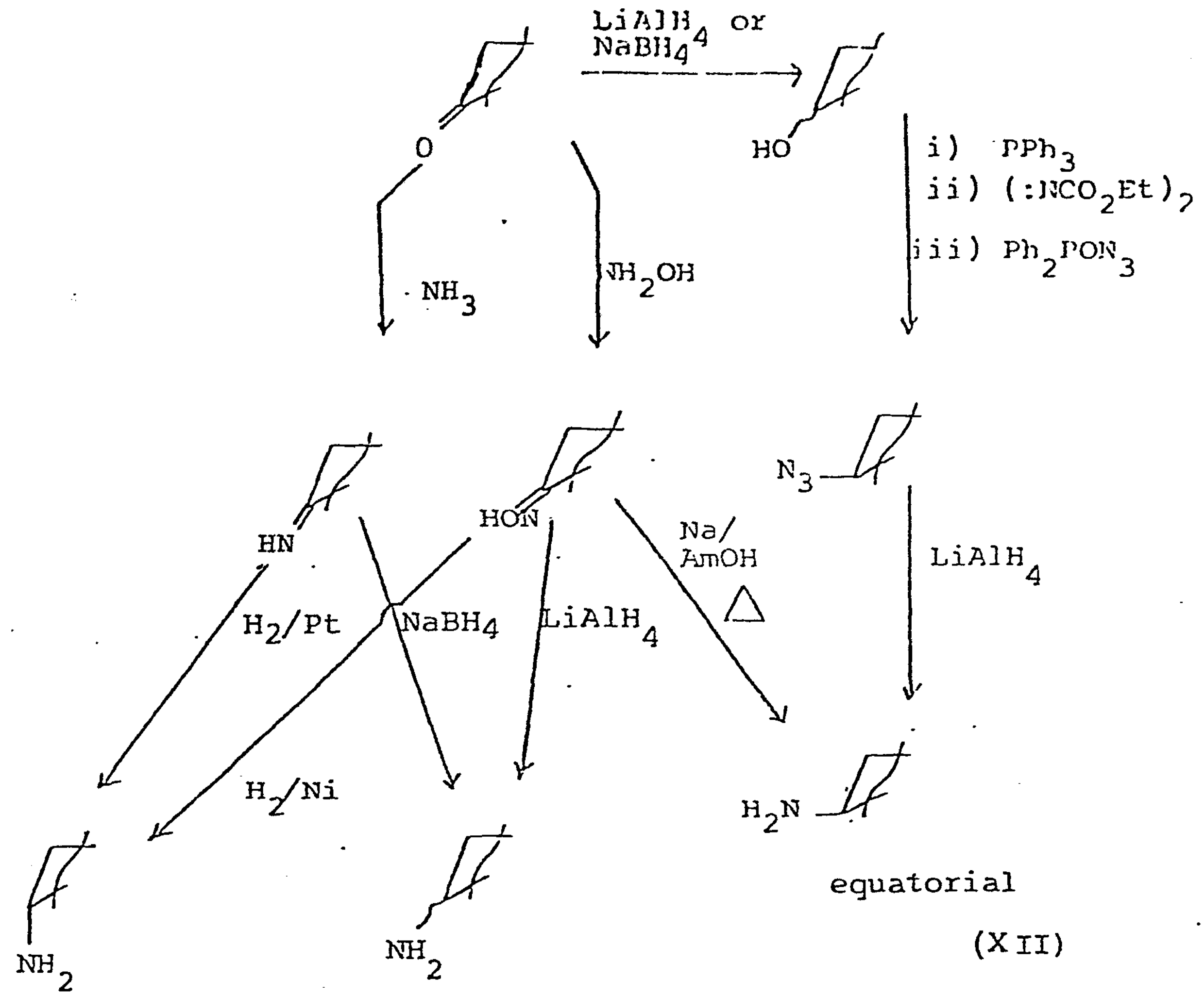


equatorial

(XII)

axial

(XII)

axial, equatorial

(XII)

(Remainder of ring system omitted for clarity)

Compounds of formula (XI) wherein $R_1$ is $C_{1-6}$ alkoxy or $C_{1-6}$ alkylthio are known or prepared by analogy with known compounds. When $R_1$ and $R_2$ together are methylenedioxy or ethylenedioxy formation of the ethylenedioxy bridge in acids of formula (XI) may be carried out by heating an acid of formula (XIV) with dibromoethane in the presence of an inorganic base such as potassium hydroxide or potassium carbonate using ethanol-water as the solvent.

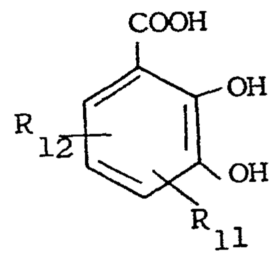

(XIV)

Compounds of the formula (XIV) are known compounds or prepared by analogy with known compounds.

As hereinbefore stated, the compounds of the formula (I) are dopamine antagonists.

Depending on their balance between peripheral and central action, the compounds of the formula (I) may be used in the treatment of disorders related to impaired gastro-intestinal motility, such as retarded gastric emptying, dyspepsia, flatulence, oesophagal reflux, peptic ulcer and emesis, and/or in the treatment of disorders of the central nervous system, such as psychosis.

All the compounds of the formula (I) may be used in the treatment of emesis.

Examples of compounds of the formula (I) which are of particular interest for their CNS activity, in particular interest for their CNS activity, in particular anti-pyschotic activity, are those of formulae (IV) and (IX).

Compounds of the formulae (IV) and (IX) also have a low level of side effects (such as extra pyramidal effects).

Examples of compounds of more interest for their beneficial effect on gastric motility are the quaternary ammonium salts of the compounds of the formula (I).

The invention also provides a pharmaceutical composition comprising a compound of the formula (I), or a solvate or a pharmaceutically acceptable salt thereof, together with a pharmaceutically acceptable carrier. Such compositions may be adapted for oral or parenteral administration, and as such may be in the form of tablets, capsules, oral liquid preparations, powders, granules, lozenges, reconstitutable powders, injectable and infusable solutions or suspensions the compositions may also be in the form of suppositories. Normally orally administrable compositions are preferred.

The invention further provides a method of treatment of maladies in mammals including humans comprising the administration of an effective amount of a compound of the formula (I) or a pharmaceutically acceptable salt thereof. The "effective amount" will depend in the usual way on a number of factors such as the nature and severity of the malady to be treated, and the actual compound used. The compositions of this invention will most suitably be presented as unit dose compositions containing from 1 to 100 mg, more usually from 2.5 to 50 mg, for example from 5 to 25 mg, such as 7.5, 10.0, 12.5 or 15 mg. Such compositions will normally be taken from 1 to 6 times daily, for example 3 or 4 times daily so that the total amount of active agent administered is within the range 2.5 to 200 mg.

Preferred unit dosage forms include tablets and capsules.

The compositions of this invention may be formulated by conventional methods of blending, filling and compressing.

Suitable carriers for use in this invention include diluents, binders, disintegrants, colouring agents, flavouring agents and preservatives. These agents may be utilized in conventional manner, for example in a manner similar to that already used for other mood modifying agents.

The following Examples illustrate the preparation of the compounds of formula (I) and the following Descriptions illustrate the preparation of the intermediates thereto.

Description 1

(a) 9-(4-methylbenzyl)-9-aza-bicyclo-[3.3.1]-nonan-3-one

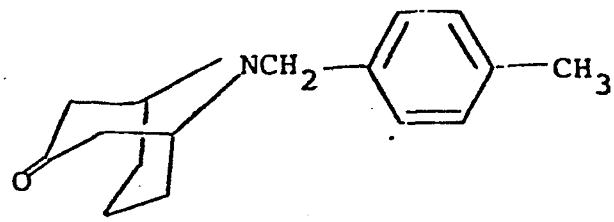

A solution of 4-methylbenzylamine (12 g) in dilute hydrochloric acid (5N, 20 ml) was added to glutaric dialdehyde (50%, 24 ml) in water (100 ml) with stirring. A solution of 1,3-acetonedicarboxylic acid (14.6 g) and sodium acetate (8.2 g) in water (100 ml) was then added and the mixture stirred for 24 hours at room temperature. A further quantity of dilute hydrochloric acid (5 ml) was then added and the mixture stirred for a further 48 hours.

It was made acidic and the acid solution washed with ether. The acidic solution was then basified and extracted with ether. The ether extract was washed with water, dried and evaporated to give the crude ketone. This was purified by filtering a solution in ethyl acetate through alumina. The yield was 13.5 g (56%).

(b) 9-(4-methylbenzyl)-9-aza-bicyclo-[3.3.1]-nonan-3-one oxime

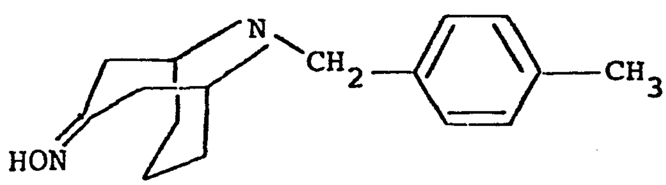

A solution of N-(4-methylbenzyl)-9-aza-bicyclo-[3.3.1]-nonan-3-one (5 g), hydroxylamine hydrochloride (1.5 g) and

pyridine (0.75 ml) in ethanol (100 ml) was heated at reflux for 1 hour. The solution was evaporated and the residue dissolved in ethyl acetate and water. It was basified with potassium carbonate and the ethyl acetate extracted, separated, dried and evaporated to give the crude oxime. Recrystallisation from ethyl acetate/light petroleum gave the oxime (4 g, 77%).

(c) <u>3β-Amino-9-(4-methylbenzyl)-9-azabicyclo[3.3.1]-nonane</u>

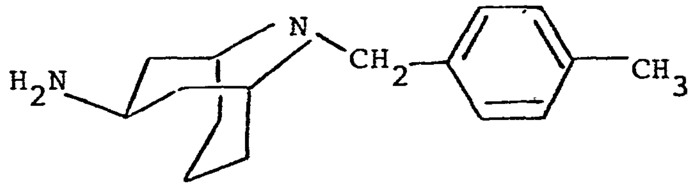

9-(4-methylbenzyl)-9-aza-bicyclo-[3.3.1]-nonan-3-one oxime (4 g) was dissolved in amyl alcohol (100 ml) and heated to reflux. Sodium (2 g) was added portionwise whilst the solution was stirred. After all the sodium had dissolved, the solution was cooled, diluted with ether and acidified with dilute hydrochloric acid. The acid extract was washed with ether and then basified with excess potassium carbonate. Extraction with ethyl acetate followed by evaporation of solvent afforded crude 3-amino-9-(4-methylbenzyl)-9-azabicyclo[3.3.1]-nonane (3.6 g, 95%) used in the next procedure without purification.

This amine is predominantly one isomer and this isomer is presumed to have the 3β-configuration.

Description 2

a) 3β-Acetylamino-9-benzyl-9-azabicyclo[3.3.1]nonane

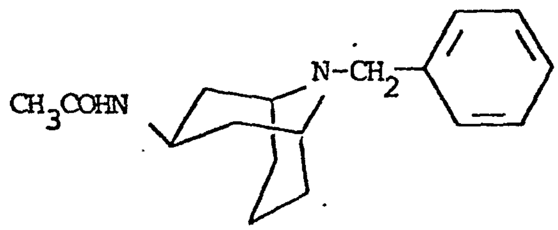

A solution of 3β-amino-9-benzyl-9-azabicyclo [3.3.1]nonane (7.2 g) and excess acetic anhydride in ethanol (100 ml) was stirred at room temperature for 48 hours. The solvent was evaporated and the residue dissolved in water and dichloromethane. It was basified with potassium carbonate and the dichloromethane extracted, separated, dried and evaporated to give 3β-acetylamino-9-benzyl-9-azabicyclo[3.3.1]nonane (6.5 g, 73%).

b) 3β-Acetylamino-9-azabicyclo[3.3.1]nonane

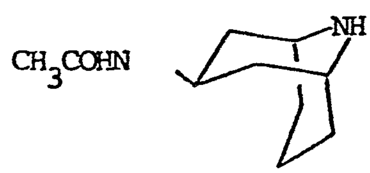

A solution of 3β-acetylamino-9-benzyl-9-azabicyclo[3.3.1]nonane (6 g) in ethanol (300 ml) was hydrogenated at room temperature and atmospheric pressure with 10% palladium on charcoal. The solution was filtered and the solvent evaporated to give the title compound (4.1 g, 100%).

c) 3β-Acetylamino-9-(4-fluorobenzyl)-9-aza-bicyclo-[3.3.1]nonane

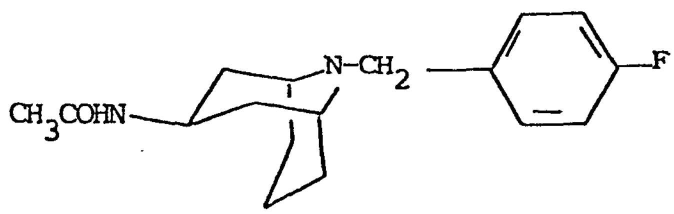

A solution of 3-acetylamino-9-azabicyclo-[3.3.1] nonane (3 g), 4-fluorobenzyl chloride (2.4 g) and potassium carbonate (4 g) in DMF (100 ml) was stirred at room temperature for 48 hours. The solution was evaporated and the residue dissolved in water. Extraction with ethyl acetate followed by evaporation of solvent afforded 3-acetylamino-9-(4-fluorobenzyl)-9-azabicyclo-[3.3.1]nonane (4.2 g, 90%).

d) <u>3β-Amino-9-(4-fluorobenzyl)-9-azabicyclo-[3.3.1] nonane</u>

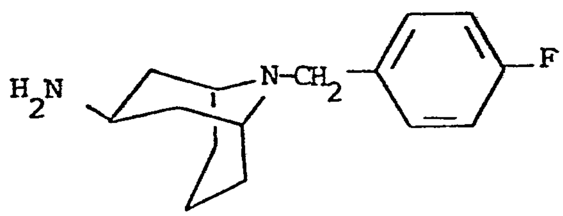

.HCl

A solution of 3-acetylamino-9-(4-fluorobenzyl)-9-azabicyclo-[3.3.1]nonane (4.2 g) in ethanol (60 ml) and concentrated hydrochloric acid (10 ml) was heated at reflux for 24 hours. The solution was evaporated and the residue dissolved in water, basified with potassium carbonate and extracted with dichloromethane. Evaporation of solvent afforded 3-amino-9-(4-fluorobenzyl)-9-azabicyclo-[3.3.1]nonane (2.6 g, 72%) which was used in the next procedure without purification.

Description 3

6-Chloro-2,3-dimethoxybenzoic acid

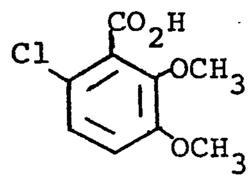

(D3)

A solution of 2,3-dimethoxybenzoic acid (5g) in glacial acetic acid (200 ml) was treated with chlorine (1 equivalent) and the solution stirred at room temperature for 2 hours. The solvent was evaporated to give 6-chloro-2,3-dimethoxybenzoic acid (5.8g, 89%) which was used in the next procedure without purification.

Description 4

(a) 5-Chloro-2-hydroxy-3-methoxybenzoic acid

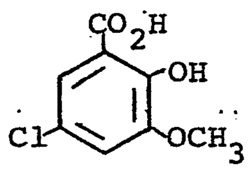

(D4)

A solution of 3-methoxysalicyclic acid (5g) in glacial acetic acid (200 ml) was treated with chlorine (1 equivalent) and the solution stirred at room temperature for 2 hours. The solvent was evaporated to give 5-chloro-2-hydroxy-3-methoxybenzoic acid (5.6g, 92%).

(b) 5-Chloro-2,3-dimethoxybenzoic acid

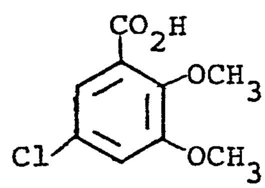

(D4)

A solution of 5-chloro-2-hydroxy-3-methoxybenzoic acid (5.6g) and dimethyl sulphate (7.2g) in acetone (100 ml), with potassium carbonate (10g), was stirred at room temperature for 24 hours. The solution was filtered and the solvent evaporated to give methyl 5-chloro-2,3-dimethoxybenzoate which was refluxed in 10% sodium hydroxide solution (50 ml) for 24 hours. The solution was cooled, acidified with dilute hydrochloric acid and filtered to give 5-chloro-2,3-dimethoxybenzoic acid (6.0g, 97%) which was used in the next stage without purification.

Example 1

2,3-Dimethoxy-N-[9-(4-methylbenzyl)-9-azabicyclo[3.3.1]nonan-3β-yl]benzamide

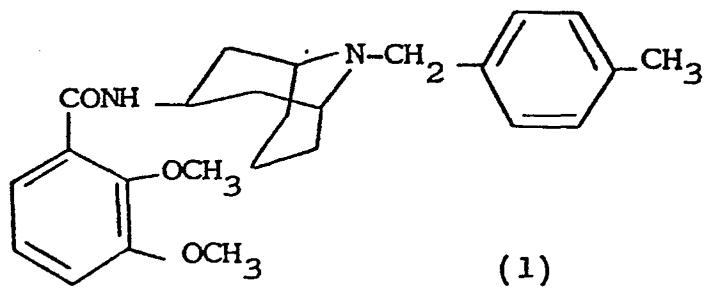

(1)

To 2,3-dimethoxybenzoyl chloride (2 g) in dichloromethane (80ml) and triethylamine (5 ml) was added 3β-amino-9-(4-methylbenzyl)-9-azabicyclo[3.3.1]nonane (2.44 g) in dichloromethane (50 ml). The reaction mixture was stirred at room temperature for 24 hours. It was then diluted with water, basified with potassium carbonate and the dichloromethane separated and dried. Evaporation of the dichloromethane gave a crude product which was recrystallised from ethyl acetate to give the title compound (2 g, 50%) mp 132-3°.

Example 2

2,3-Dimethoxy-N-[9-(4-fluorobenzyl)-9-azabicyclo[3.3.1]nonan-3β-yl]benzamide

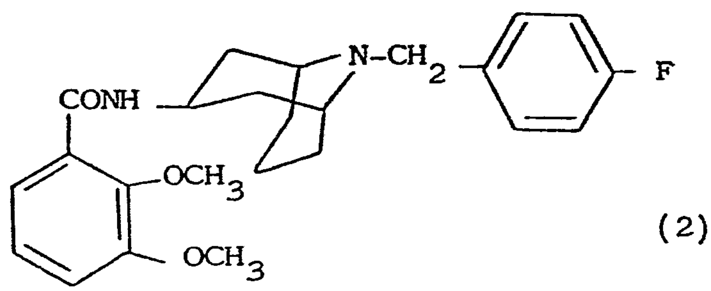

(2)

To 2,3-dimethoxybenzoyl chloride (1g) in dichloromethane (50 ml) and triethylamine (3 ml) was added 3β-amino-9-(4-fluorobenzyl)-9-azabicyclo[3.3.1]nonane (1.24 g) in dichloromethane (25 ml). The reaction mixture was stirred at room temperature for 24 hours. It was then diluted with water, basified with potassium carbonate and the dichloromethane separated and dried. Evaporation of the dichloromethane gave a crude product which was recrystallised from ethyl acetate to give the title compound (1.1 g 52%) mp 135-7$^{o}$.

Example 3

6-Chloro-2,3-dimethoxy-N-[9-(4-fluorobenzyl)-9-azabicyclo[3.3.1]nonan-3β-yl]benzamide

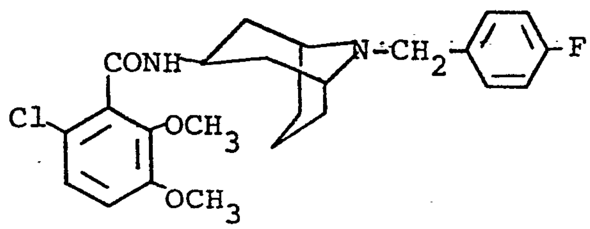

(E3)

The title compound (3) m.p. 169-171$^{\circ}$, was prepared in 60% yield from 6-chloro-2,3-dimethoxybenzoyl chloride as described for Example 1.

Example 4

5-Chloro-2,3-dimethoxy-N-[9-(4-fluorobenzyl)-9-azabicyclo [3.3.1]nonan-3β-yl]benzamide

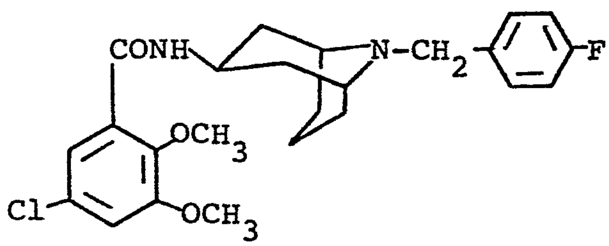

(E4)

The title compound (4), m.p. 152-154° was prepared in 30% yield from 5-chloro-2,3-dimethoxybenzoyl chloride as described for Example 1.

PHARMACOLOGICAL DATA

The results in the following table are an illustration of the anti-psychotic activity of the present compounds as shown by Inhibition of Apormorphine Induced Climbing in the Mouse, a standard test.

Inhibition of apomorphine induced climbing in the mouse

The test is based on that described by Protais, P., Constantin, J. and Schwartz J.C. (1976), Psychopharmacology, 50, 1-6.

When mice are given a dose of 1 mg/kg apomorphine and then placed in an enclosed environment, such as an inverted wire cage, they are seen to climb around the walls. This behavioural phenomenon is thought to be a consequence of the stimulation of post-synaptic Dopamine (D.A.) receptors in the nucleus accumbens. Inhibition of apomorphine induced climbing is therefore indicative of post-synaptic D.A. receptor blockade in the accumbens.

Groups of 10 male CD1 mice, weighing 25-30 g were pretreated orally with either graded doses of the test compound or vehicle, at 30 minutes before the subcutaneous administration of a sub-maximal dose of apomorphine (1 mg/kg). Immediately after the apomorphine injection the mice were placed in wire 'climbing cages' and each animal was scored for climbing behavour at 10 and 20 minutes post apomorphine as follows:-

Four paws on cage floor = 0
Fore paws on cage wall = 1
Four paws on cage wall = 2

The total score was calculated for each group of mice and expressed as a percentage inhibition of climbing.

$$\% \text{ inhibition} = 100 - \frac{\text{Total score for test compound}}{\text{Total score for apomorphine control}} \times 100$$

ED50's and fiducial limits were calculated according to the method of Litchfield and Wilcoxon, the ED50 being the dose that produced a 50% inhibition of apomorphine-induced climbing.

The table shows the dose for 50% inhibition.

| Compound | $ED_{50}$ mg/kg |
|---|---|
| (1) | 0.19 |
| (2) | 0.12 |
| (3) | 3.4 |
| (4) | 0.67 |

Toxicity

No toxic effects were observed in the above test.

0083737

Claims

1. A compound of formula (I) or a pharmaceutically acceptable salt thereof:

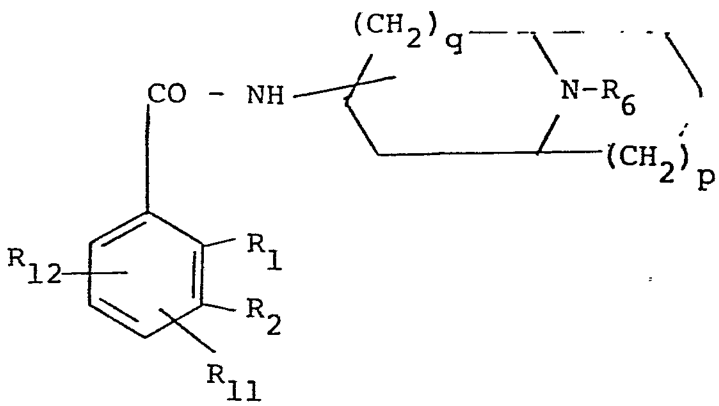

(I)

wherein:

p is 1 or 2;

q is 0 to 2;

$R_6$ is $C_{1-7}$ alkyl or a group $-(CH_2)_sR_7$ where s is 0 to 2 and $R_7$ is a $C_{3-8}$ cycloalkyl group, or a group $-(CH_2)_tR_8$ where t is 1 or 2 and $R_8$ is $C_{2-5}$ alkenyl or a phenyl group optionally substituted by one or two substituents selected from $C_{1-4}$ alkyl, $C_{1-4}$ alkoxy, trifluoromethyl, halogen or nitro, or a thienyl group;

$R_{12}$ is hydrogen, halogen, $CF_3$, $C_{1-7}$ acyl, $C_{1-7}$ acylamino or amino or aminocarbonyl optionally substituted by one or two $C_{1-6}$ alkyl groups, $C_{1-6}$ alkyl, $C_{1-6}$ alkoxy, hydroxy or nitro;

$R_1$ is $C_{1-6}$ alkoxy or $C_{1-6}$ alkylthio; and one of $R_2$ and $R_{11}$ is hydrogen and the other is $C_{1-6}$ alkoxy, $C_{1-6}$ alkyl or hydroxyl;

or $R_1$ and $R_2$ together form methylenedioxy or ethylenedioxy; and

$R_{11}$ is any one of the groups given for $R_{12}$ above.

2. A compound according to claim 1 of the formula (IV):

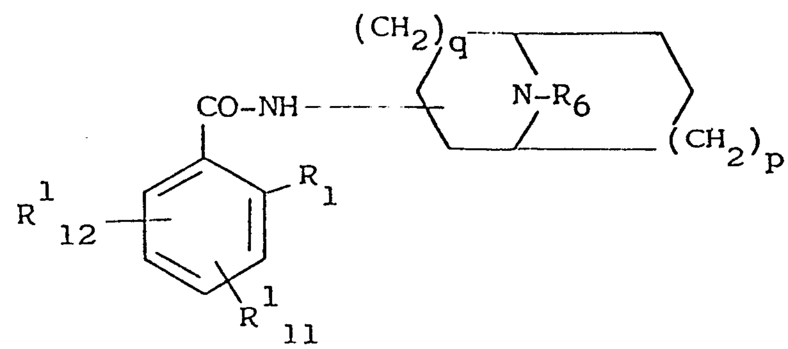

(IV)

wherein:

$R_1{}^1$ is $C_{1-6}$ alkoxy or $C_{1-6}$ alkylthio;

one of $R_{11}{}^1$ and $R_{12}{}^1$ is $C_{1-6}$ alkoxy and the other is $C_{1-6}$ alkoxy or hydrogen; and the remaining variables are as defined in claim 1.

3. A compound according to claim 1 or 2 of the formula (VI):

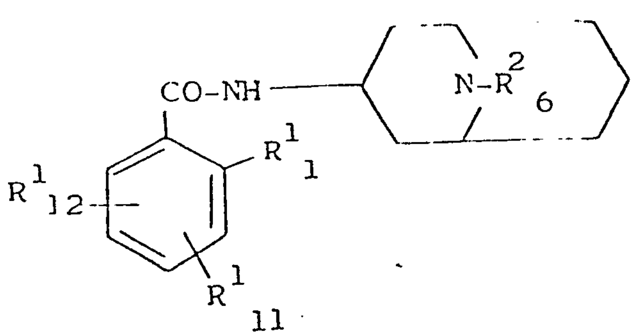

(VI)

wherein:

$R_6{}^2$ is a group $-(CH_2)_tR_7{}^1$ wherein t is 1 or 2 and $R_7{}^1$ is optionally substituted phenyl as defined in formula (I); cyclohexylmethyl; or 2-thienylmethyl; and the remaining variables are as defined in claim 2.

 0083737

4. A compound according to claim 3 of the formula (VII):

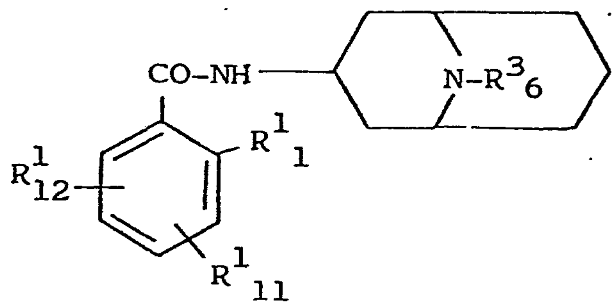

(VII)

wherein $R_6^3$ is benzyl <u>para</u> substituted by $C_{1-4}$ alkyl or halogen and the remaining variables are as defined in claim 2.

5. A compound according to any one of the claims 2 to 4 wherein $R_1^1$ and $R_{11}^1$ are both methoxy and $R_{12}^1$ is hydrogen.

6. A compound according to claim 1 of the formula (IX):

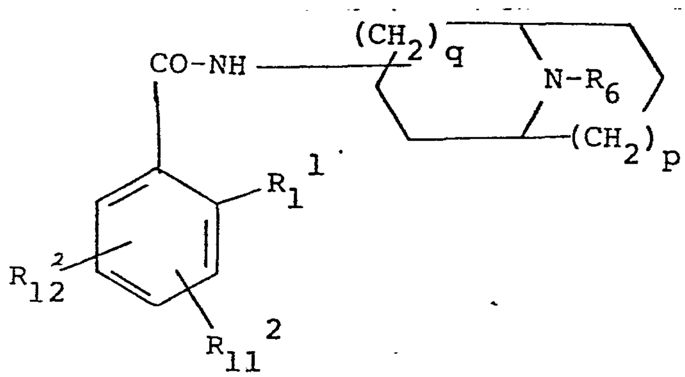

(IX)

wherein one of $R_{11}^2$ and $R_{12}^2$ is $C_{1-6}$ alkoxy and the other is halogen or $CF_3$; and the remaining variables are as defined in claims 1 and 2.

7. A compound according to claim 6 of the formula (X):

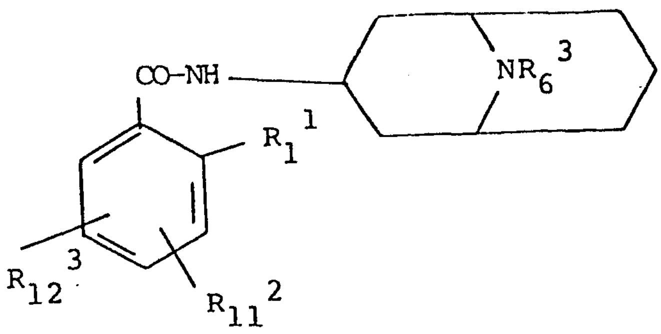

(X)

wherein the variable groups are as defined in claims 4 and 6.

8. A compound according to claim 6 or 7 wherein $R_1{}^1$ and $R_{11}{}^2$ are both methoxy and $R_{12}{}^2$ is chloro or bromo.

9. A compound according to claim 4, 5, 7 or 8 wherein $R_6{}^3$ is p-methylbenzyl, p-chlorobenzyl or p-fluorobenzyl.

10. 2,3-Dimethoxy-N-[9-(4-methylbenzyl)-9-azabicyclo-[3.3.1]nonan-3β-yl]benzamide, 2,3,-Dimethoxy-N-[9-(4-fluorobenzyl)-9-azabicyclo[3.3.1]nonan-3β-yl]-benzamide, 6-Chloro-2,3-dimethoxy-N-[9-(4-fluorobenzyl)-9-azabicyclo[3.3.1]nonan-3β-yl]benzamide or 5-Chloro-2,3-dimethoxy-N-[9-(4-fluorobenzyl)-9-azabicyclo[3.3.1]nonan-3β-yl]benzamide.

11. A process for the preparation of a compound according to any one of the claims 1 to 10 which process comprises reacting a compound of the formula (XI):

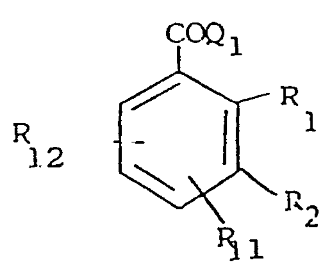

(XI)

wherein $Q_1$ is a leaving group with a compound of formula (XII):

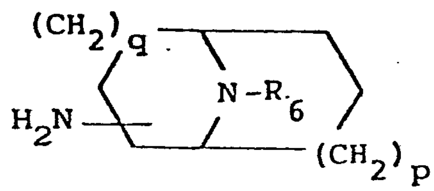

(XII)

wherein the variables are as defined in claim (I), and thereafter if desired or necessary converting a group $R_2$, $R_{11}$ or $R_{12}$ in the thus formed compound to converting $R_6$ to (other) $R_6$; and optionally forming a pharmaceutically acceptable salt of the resultant compound of the formula (I).

12. A pharmaceutical composition comprising a compound according to any one of the claims 1 to 10 or a solvate or a pharmaceutically acceptable salt thereof, and a pharmaceutically acceptable carrier.

13. A compound according to any one of the claims 1 to 10 for use in the treatment of disorders of the central nervous system.

European Patent Office

# EUROPEAN SEARCH REPORT

0083737

Application number

EP 82 11 1332

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | GB-A-2 042 522 (DELALANDE)<br><br>*Claims; examples 51,57,60,61,64,68*<br>--- | 1-5,7-9,11-13 | C 07 D 451/14<br>C 07 D 451/04<br>A 61 K 31/435//<br>C 07 C 65/21 |
| Y,D | EP-A-0 013 138 (BEECHAM)<br>*The whole document*<br>--- | 1-13 | |
| P,X | GB-A-2 088 364 (DELALANDE)<br><br>*The whole document*<br>----- | 1-5,7-9,11-13 | |
| | | | TECHNICAL FIELDS SEARCHED (Int. Cl. 3) |
| | | | C 07 D 451/00<br>A 61 K 31/00 |

The present search report has been drawn up for all claims

| Place of search | Date of completion of the search | Examiner |
|---|---|---|
| THE HAGUE | 27-04-1983 | NUYTS A.M.K.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82